# EUROPEAN PATENT SPECIFICATION

(11) **EP 1 737 511 B1**
(45) Date of publication and mention of the grant of the patent: **01.09.2010**
(21) Application number: 05724194.5
(22) Date of filing: 02.03.2005
(51) Int. Cl.: A61M 5/175, G05D 7/01, F15D 1/10

(54) **FLOW RESTRICTOR DEVICE FOR A MEDICAL APPARATUS**
DROSSELGERÄT FÜR EINEN MEDIZINISCHEN APPARAT
DISPOSITIF REDUCTEUR DE DEBIT POUR APPAREIL MEDICAL

(30) Priority: 02.03.2004 US 791682; 02.08.2004 US 909752
(43) Date of publication of application: 03.01.2007
(73) Proprietor: Schinazi, Robert G., Vista CA 92081 (US); De Rosset, Lauren E., Vista CA 92081 (US)
(72) Inventor: Schinazi, Robert G., Vista CA 92081 (US); De Rosset, Lauren E., Vista CA 92081 (US)
(74) Representative: Schlief, Thomas P.
(86) International application number: PCT/US2005/006600
(87) International publication number: WO 2005/084310

(56) References cited:
- EP-A- 0 271 785
- GB-A- 376 798
- US-A- 2 341 394
- US-A- 2 511 733
- US-A- 2 771 878
- US-A- 3 815 636
- US-A- 4 878 649
- US-A- 5 163 920
- US-B1- 6 550 956

## Description

### FIELD OF THE INVENTION

The present invention relates generally to the field of flow restriction devices, and more specifically to a flow restrictor that is particularly well suited for regulating the flow of fluids in various medical devices and systems.

### BACKGROUND OF THE INVENTION

There are various applications of flow restriction devices in the medical arts for closely regulating the flow of a fluid. One common use of such devices is with an infusion pump system wherein fluid medicine or other fluids are delivered to an injection site on the patient from an infusion pump. Embodiments are known wherein the flow restrictor is contained within the pump body. For example, U.S. Pat. No. 4,386,929 describes a short capillary tube contained within the pump housing for regulating the flow of dispensed medication. It is also known to include a flow restrictor downstream of the infusion pump, for example as with the delivery tube system described in U.S. Pat. No. 4,741,733.

U.S. Pat. No. 6,569,128 describes a catheter flow restriction system wherein a capillary-like restriction tube is contained within a catheter tube. The flow rate through the system is adjusted by trimming the length of the restriction tube and concentric catheter tube. The catheter can then be attached to an infusion device by a suitable connector, such as a Touhy-Borst connector.

Another throttling device, which is not designed to be used as a medical apparatus flow restrictor but comprises the combination of features of the preamble of claim 1, is described in U.S. Pat. No. 2,341,394. An adjustable cylindrical plug with a roughened surface is axially movable within a discharge piece. Here, the liquid flows through a clearance of, e.g., 0,5 mm between said cylindrical plug and the inside of said discharge piece.

Conventional flow restriction devices are, however, not without certain drawbacks, particularly the capillary tube restrictors. For example, such tube-type devices are relatively difficult and expensive to manufacture. Also, as requirements in the medical field tend towards decreased flow rates, it has become increasingly difficult to manufacture the tubes to achieve a specified flow rate due simply to machining tolerances and material limitations. For example, the smaller (in diameter) the tubes become, the more prone they become to particulate clogging.

### SUMMARY OF THE INVENTION

Objects and advantages of the invention will be set forth in part in the following description, or may be obvious from the description, or may be learned through practice of the invention.

In accordance with the invention, which is defined in claim 1, a flow restrictor device is provided that is relatively inexpensive to manufacture and assemble, yet will reliably maintain a precisely regulated flow rate. The device is not prone to clogging, and is easily incorporated into any conventional medical infusion or other type of fluid delivery system. In this regard, it should be appreciated that although the inventive restrictor has particular usefulness in an infusion delivery system, the invention is not limited to this particular application. The inventive restrictor may be used in any system wherein it is desired to restrict or regulate the flow of a fluid, and all such uses are within the scope and spirit of the invention.

The term "fluid" is used herein to refer to a gas, liquid, or combination of a gas and liquid.

The flow restrictor according to the invention includes a housing having an inlet and an outlet, and a fluid path defined through the housing between the inlet and outlet. The inlet and outlet are configured to be connected in-line, for example with tubing or the like, in a medical apparatus, such as an infusion system. The inlet and outlet may be releasably connected to the medical apparatus tubing, for example with a simple press fit, clamp, or fitting, or permanently attached by, for example, by way of an adhesive, ultrasonic bond, a weld, and so forth.

At least one pair of opposed restriction devices are seated within the housing between the inlet and outlet. The restriction devices have opposing surfaces placed in contact against each other and are disposed in the flow path such that fluid delivered to the inlet must pass between the opposing surfaces prior to flowing from the outlet. Any one or combination of seals, such as O-rings, gaskets, or the like, may be used within the housing to establish the desired flow path through the devices. A resilient member, such as a spring, wave spring, or similar device may be used to bias the restriction devices together. Alternatively, the seal may be resilient and also function to bias the restriction devices together.

The opposing surfaces of the restriction devices have a relative degree of surface roughness and opposed surface area that are predetermined as a function of a desired flow rate of fluid through the restrictor. Thus, the restrictive flow path between the opposed surfaces of the restriction devices has a metering or restrictive effect on the rate of flow through the device, as described in greater detail below.

The restriction devices may take on various shapes and be formed from any number of suitable materials, such as glass, ceramic, steel, and so forth. For example, in one particular embodiment, the restriction devices are opposed flat planar members disposed within the housing such that fluid from the inlet flows radially between the opposing surfaces. In one particular embodiment, fluid from the inlet is directed to the outer circumference of the restriction devices and flows radially inward between the opposing surfaces. The bottom (downstream) member has an orifice defmed therethrough that defines an exit path for the fluid from between the planar members. The orifice is aligned with, or otherwise in fluid communication with, the housing outlet.

In an alternate embodiment, the upstream restriction device (e.g., an upstream flat planar member) may have an opening or orifice and the flow path within the housing is established such that fluid flows through this orifice and then migrates radially outward between the opposing surfaces prior to flowing to the outlet.

The flat planar member restriction devices may take on various shapes, sizes, thicknesses, etc. In one particular embodiment, the members are circular discs stamped or otherwise formed from a desired material. Such devices may be desired from the standpoint of ease of manufacture and assembly.

It should be appreciated that it is not necessary that each of the opposing surfaces is purposefully roughened as compared to the other. A desired relative degree of surface roughness along the restrictive flow path may be achieved by treating only one of the surfaces. The other surface may be untreated and relatively smooth. Alternately, the surfaces may have an inherent degree of surface roughness such that neither surface need be treated.

In an alternate embodiment, the restriction devices may be defined by a conical male member that mates within a complimentary shaped recess such that the opposing surfaces are defined by the conical wall of the male member and the recess wall. The conical member may have straight sides (i.e., constant slope) or curved sides. This embodiment may be desired in that a larger surface area between the opposing surfaces of the restriction devices may be achieved, thus permitting a greater degree of metering or fluid restriction.

In yet another embodiment, the restriction devices may be defined by a ball member seated within a ball seat such that the opposing surfaces are defined by a circumferential portion of the ball member and the ball seat. One or both of these surfaces may be roughened.

The restriction devices may be formed of a hard, non-compressible material, such as a medical grade stainless steel, so that fluid flow between the opposing surfaces is substantially constant regardless of a compressive pressure applied to restriction devices from fluid pressure or assembly of the housing components. In an alternate embodiment, the restriction devices may be formed of a compressible material, such as a medical grade polymer material, so that fluid flow between the opposing surfaces may be changed or adjusted by varying a compressive pressure applied to the restriction devices, for example by way of housing components that may be threadedly engaged.

In a particular embodiment, the housing comprises separate halves, with the restriction devices being placed within the halves prior to joining the halves to form the complete housing. The halves may be separable after being joined for access to the restriction devices. For example, the halves may be threaded onto each other, or otherwise releasably engaged. Alternatively, the halves may be permanently joined, for example by way of an adhesive, weld, and so forth.

The restriction devices may be variously oriented within the housing relative to the inlet and outlet. For example, in one embodiment, the devices are disposed such that a plane between the opposing surfaces is generally perpendicular to an axis of the inlet and outlet. In an alternate embodiment, the restriction devices are disposed such that the plane between the opposing surfaces is generally parallel to an axis of the inlet and outlet.

The invention also encompasses any manner of medical fluid delivery system that incorporates one or more of the unique fluid restriction devices as described herein.

The invention will be described in greater detail below by reference to particular embodiments shown in the referenced figures.

### BRIEF DESCRIPTION OF THE FIGURES

Fig. 1A is a perspective view of a conventional infusion pump system incorporating a flow restrictor according to the invention.

Fig. 1B is a perspective view of a conventional portable medical infusion system incorporating a flow restrictor according to the invention.

Fig. 1C is a perspective view of yet another conventional medical infusion system incorporating a flow restrictor according to the invention.

Fig. 2 is a perspective view of an embodiment of a flow restrictor according to the invention.

Fig. 3A is a cross-sectional view of the flow restrictor of Fig. 2, particularly illustrating the flow path for a fluid through the device.

Fig. 3B is an enlarged view of the section of the circumference of the flow restriction devices indicated in Fig. 3A.

Fig. 3C is a side view of an embodiment of compressible flow restriction devices.

Fig. 4A is an in-line component view of an embodiment of the flow restrictor particularly illustrating the fluid flow path from the inlet to the outlet.

Fig. 4B is an in-line component view of the embodiment of Fig. 4A taken from the opposite direction.

Fig. 5 is a perspective view of an alternate embodiment of a flow restrictor according to the invention.

Fig. 6 is a component view of the embodiment of Fig. 5.

Fig. 7 is a perspective and partial cut-away view of an alternative embodiment of a flow restrictor according to the invention utilizing conical restriction devices.

Fig. 8 is a perspective and partial cut-away view of an alternative embodiment of a flow restrictor according to the invention utilizing conical restriction devices having radially curved side walls.

Fig. 9 is a cross-sectional diagrammatic view of an alternate embodiment of a flow restrictor incorporating a biasing element with the housing.

Fig. 10 is a cross-sectional diagrammatic view of an embodiment of a flow restrictor having a flow path such that fluid flows radially outward between the opposed surfaces of the restriction devices.

Fig. 11 is a cross-sectional diagrammatic view of an embodiment of a flow restrictor wherein the restriction devices include a ball seated within a ball seat.

### DETAILED DESCRIPTION

Reference will now be made in detail to examples of the invention, one or more embodiments of which are illustrated in the figures. Each example is provided by way of explanation of the invention, and not meant as a limitation of the invention. For example, features illustrated or described as part of one embodiment may be used with another embodiment to yield still a further embodiment. It is intended that these and other modifications and variations be included within the scope and spirit of the invention.

Fig, 1A illustrates an embodiment of a medical system, generally 12, that may utilize a flow restrictor 10 according to the invention. The medical system 12 is illustrated as a conventional infusion system wherein an infusion pump 14 is supplied with a fluid, such as a medicine, by a solution container 16 that is supported at a given height above the pump 14. Tubing 20 supplies the fluid from the pump 14 to an intravenous (IV) site 22 on a patient 18. Such infusion systems and pumps 14 are well known by those skilled in the medical art. Such systems are supplied, for example, by Braun Medical, Inc. of Bethlehem, PA, and Baxter Healthcare Corporation of Round Lake, Illinois. The flow restrictor 10 is illustrated as connected in-line in the tubing 20 between the pump 14 and the patient 18. It should also be appreciated that the restrictor 10 may be incorporated within the housing of the pump 14.

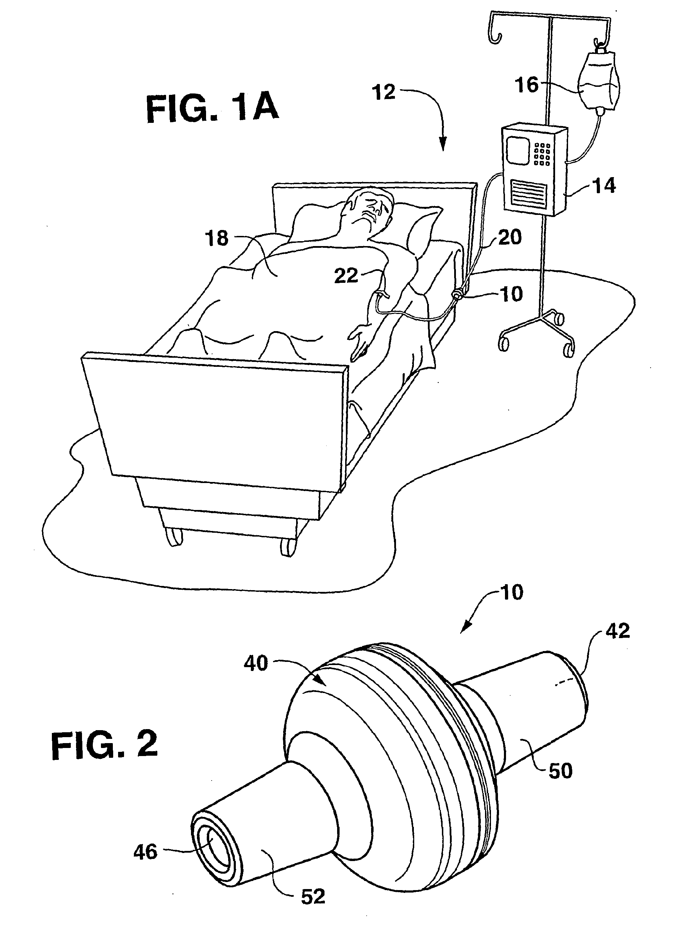

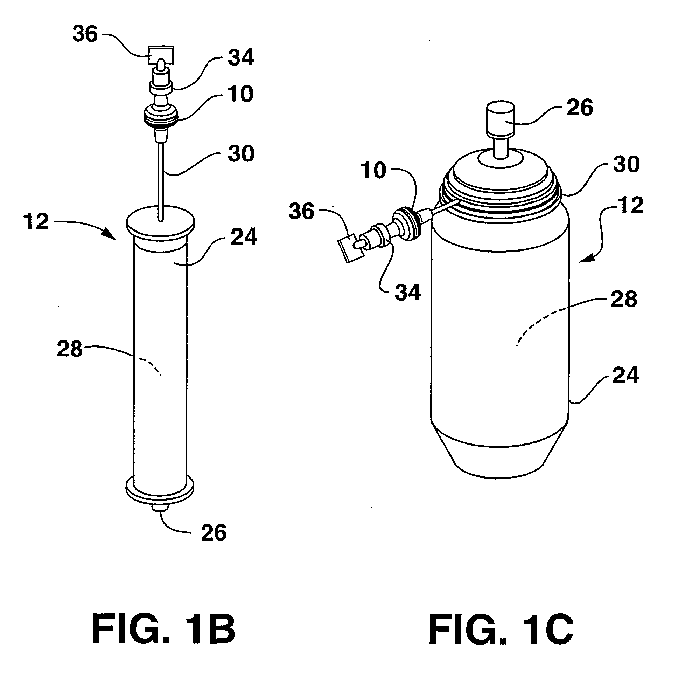

Figs. 1B and 1C illustrate portable infusion systems that are generally worn or carried by a patient. Such devices are commercially available, for example, from Baxter Healthcare Corporation. Fig. 1B illustrates a small-volume system wherein a housing 24 defines an internal reservoir 28. A flexible membrane, such as an elastomeric balloon or the like, is contained within the reservoir 28 and provides fluid pressure. A fill port and associated cap 26 are provided for filling the reservoir 28. Delivery tubing 30 connects the housing 24 to a delivery end connector 34, such as a conventional luer connector. A cap 36 is provided for the connector 34. A flow restrictor 10 in accordance with the invention is provided in-line in the tubing 30 between the connector 34 and the housing 24. The connector 10 may be removably connected in the tubing 30, or permanently attached in the tubing 30, as described in greater detail below.

The infusion device of Fig. 1C is similar to that of Fig. 1B, but includes a larger housing 24 and internal reservoir 28. The reservoir 28 also includes an elastomeric "balloon" type of member for holding the fluid medication under sufficient pressure for delivery to the patient.

Although described with reference to infusion-type systems, it should be readily appreciated that the flow restrictor 10 according to the invention may be used in any medical system wherein it is desired to deliver a metered amount of a fluid to a patient from a pressurized source. For example, the restrictor 10 according to the invention can be utilized for continuous or intermittent delivery of fluids through clinically acceptable routes of administration, such as intravenous (IV), intra-arterial (IA), subcutaneous, epidural, or irrigation of fluid spaces applications.

Fig. 2 illustrates an embodiment of the flow restrictor 10 in accordance with the invention. The restrictor 10 includes a housing 40 that may take on generally any desired shape or design. The housing 40, in one particular embodiment, is defined by separate halves or elements 50, 52, as described in greater detail below. The flow restrictor 10 includes an inlet 42 through which fluid is introduced into the device 10, and an outlet 46 from which fluid is conducted after flowing through the device 10.

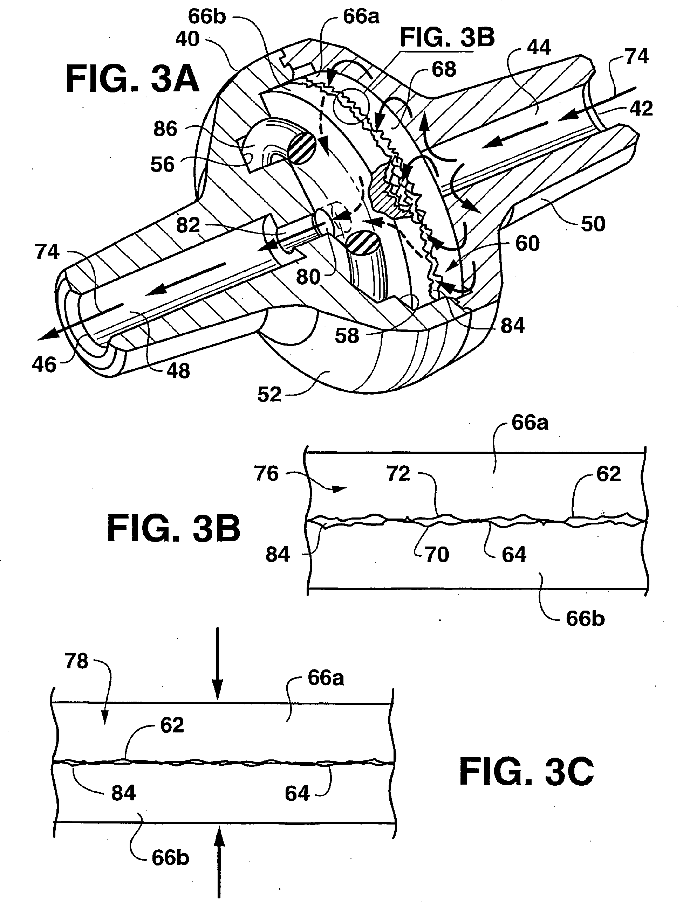

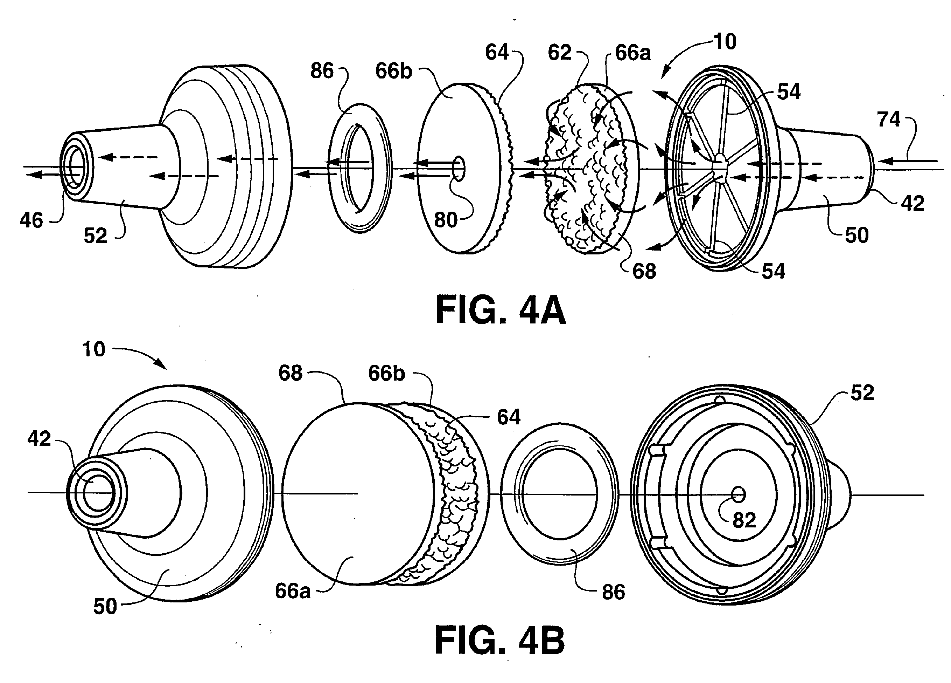

Referring to Figs. 3A through 3C, 4A, and 4B, the flow restrictor 10 of Fig. 2 is illustrated in greater detail. Housing 40 includes a first half 50 and a second half 52. The first half 50 defines the inlet 42 and an inlet passage 44. Similarly, the second half 52 defines an outlet passage 48 and the outlet 46. The halves 50, 52, may be configured to be releasably attached to medical tubing, for example by way of a clamp, or a simple friction or press fit of the tubing over the elongated ends of the respective halves 50, 52. Alternately, the tubing may be permanently attached to the respective halves 50, 52, by an adhesive, weld, or any other suitable permanent attachment means.

At least one pair of opposed restriction devices 60 are seated within the housing 40 between the inlet 42 and the outlet 46. The restriction devices 60 may take on various forms, so long as they define opposed surfaces placed in contact against each other such that a restrictive flow field 84 is defined between the opposed surfaces. For example, in the embodiment illustrated in the figures, the restriction devices 60 are defined by generally flat planar members 66a and 66b disposed against each other. The planar members may take on any shape, and in the figures are depicted as circular disks contained in the housing 40 by way of disk seats 58. Referring particularly to Figs. 3A and 3B, it can be seen that at least one of the disks 66a or 66b, includes a generally "rough" surface such that when the opposing surfaces 62 and 64 are placed against each other, the restrictive fluid flow field 84 is defined between the surfaces. In the illustrated embodiment, each of the disks includes a roughened surface, as particularly seen in Fig. 3B. In this manner, there exists void spaces or valleys 72 and protrusions 70 that define a completely random restrictive flow field 84. It should be appreciated that the degree of surface roughness between the opposing surfaces 62, 64, is grossly exaggerated in the figures for purposes of illustration only. It may very well be that, for many embodiments, the surface roughness is not discernible by the unaided eye.

By carefully controlling the degree of surface roughness of the opposed surfaces 62, 64, the rate of fluid flow between the matrix of valleys 72 and protrusions 70 along the field 84 may be controlled, and a relatively precise metering mechanism is defined. For a specific fluid pressure, a desired fluid flow rate can be achieved by carefully defining the particular parameters of surface roughness of each of the surfaces 62, 64, the surface area of contact between the surfaces 62, 64, and the pressure applied to the surfaces 62, 64. Taking these factors into consideration, the restriction devices 66a and 66b may be designed for a particular flow rate based on prediction algorithms. Alternatively, the dimensions and surface roughness of the devices 66a, 66b, may be empirically determined through routine experimentation.

The cylindrical disk-type restriction devices 66a, 66b, may be desired in that they are relatively inexpensive and easy to fabricate. For example, the components may be punched, stamped, turned, and so forth. Also, the desired degree of surface roughness of the disks 66a, 66b, may be achieved with conventional processes such as etching, sandblasting, lapping, grinding, tumbling, and so forth.

In the embodiment illustrated in Figs. 3A and 3B, the restriction devices 66a, 66b, are formed from a relatively incompressible, hard material, such as stainless steel, glass, ceramic, and so forth. In this manner, the restrictive flow path 84 is predominantly unchanged or unaltered by the degree of compression of the devices against each other. Alternatively, it may be desired that the devices 66a, and 66b, are formed from a compressible material, such as a relatively soft poly material. In this manner, the restrictive field 84 may be made more or less restrictive depending upon the degree of compression of the components 66a, 66b, against each other, as is diagrammatically illustrated in Fig. 3C. The "soft" disks 66a, 66b, may be used in an embodiment wherein the housing halves 50, 52, are relatively adjustable relative to each other, for example as in a threaded engagement between the two halves. With this configuration, different flow rates may be achieved with a single restrictor 10, or the restrictor may be adjusted or fine-tuned for achieving a very accurate flow rate.

Figs. 3A and 4A illustrate the flow path 74 of a fluid through one embodiment of the restrictor 10. In this particular embodiment, the fluid moves under pressure through the inlet 42 and inlet passage 44 and is directed to the perimeter or circumference 68 of the restriction devices 66a, 66b, by way of relief channels 54 or other suitable structure defined in the housing half 50. A sealing device 86, such as a conventional O-ring, gasket, or any other suitable elastomeric sealing device, is disposed in a seat 56 in the housing second half 52, as particularly seen in Fig. 3A. This seal 86 prevents the fluid from bypassing the restrictive field 84. Referring particularly to Fig. 3A, it can be seen that the fluid migrates from the circumference of the restriction devices 66a, 66b radially inward at a rate that is defined as a function of the surface roughness and surface area of the opposed surfaces 62, 64, of the restriction devices, as discussed above. The fluid migrates to an orifice 80 defined in the downstream restriction device 66b. The orifice 80 is in fluid communication with the outlet passage 48, for example by way of an orifice passage 82.

It should be appreciated that any number of configurations of internal structure, sealing devices, and so forth, may be utilized within a housing 40 to ensure that fluid is directed through the restrictive field 84 of opposed restriction devices 66a, 66b to disburse the fluid from the outlet 46 at a desired flow rate. It may be desired to incorporate the sealing element in the housing, for example by way of a two-shot injection molding process wherein the second shot is an elastomer. Alternatively, a sealing element may be provided on one or both of the disks 66a, 66b (or other type of restriction devices). For example, the disks 66a, 66b may be stamped from a composite metal/rubber sheet wherein the metal component defines an opposing surface of the restriction device, and the rubber component defines the seal. In still an alternative embodiment, the seal need not be elastomeric. For example, the seal may be defined by an epoxy, glue, or ultrasonic bond between the disk and a housing member.

Thus far, the restrictor 10 according to the invention has been described with opposed roughened surfaces 62, 64. However, it should be appreciated that the invention also includes the configuration wherein only one of the surfaces 62, 64, is roughened. In other words, the restrictive flow field 84 may be achieved by opposed surfaces wherein one of the surfaces is relatively smooth or polished with respect to the other surface. Alternatively, for ease of manufacturing, assembly, and so forth, it may be desired that all of the components are essentially the same and axis-symmetric. For example, if both sides of the restriction disk 66a, 66b are treated (rough), then assembly is facilitated by eliminating a particular surface to surface orientation. This design is optimized for pick-and-place automated assembly.

It should also be appreciated that, for varying flow rates, several restrictive devices may be stacked within a common housing.

The halves of the housing 40 may be releasably attached to each other after insertion of the sealing device 86 and restriction devices 66a, 66b, or permanently attached to each other. For example, the halves, 50, 52, may be threadedly engaged such that the device 10 may be subsequently taken apart for replacement of the restriction devices 66a, 66b. In an alternative embodiment, the halves 50, 52 may be permanently adhered to each other with an adhesive, ultrasonic bonding, welding, or any other conventional attaching means.

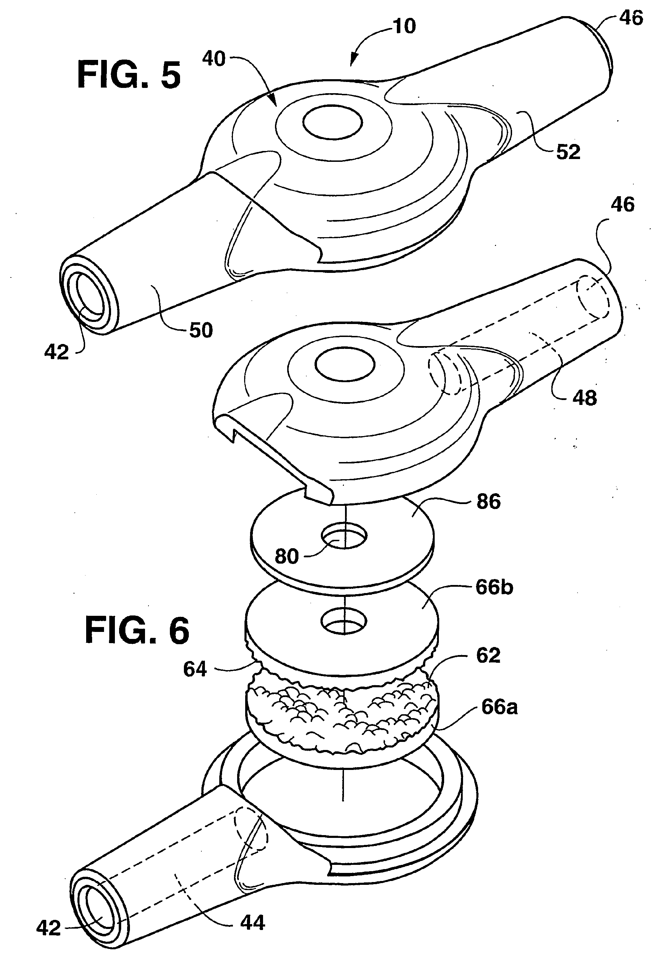

It should also be appreciated that the restriction devices 66a, 66b, may be variously oriented within the housing 40. For example, in the embodiment illustrated in Figs. 3A, 4A, and 4B, the devices 66a and 66b are oriented such that a plane between the opposing surfaces 62, 64, is generally perpendicular to the axis of the inlet and outlet of the housing 40. Figs. 5 and 6 illustrate an alternative embodiment wherein the restriction devices 66a, 66b, and seal 86 (gasket) are oriented within the housing 40 such that the plane between the restriction devices is generally parallel to the axis of the inlet and outlet of the housing 40. Regardless of the orientation of the restriction devices 66a, 66b, the operation of the device is essentially as described above with reference to the embodiment of Figs. 3A and 4A, and 4B.

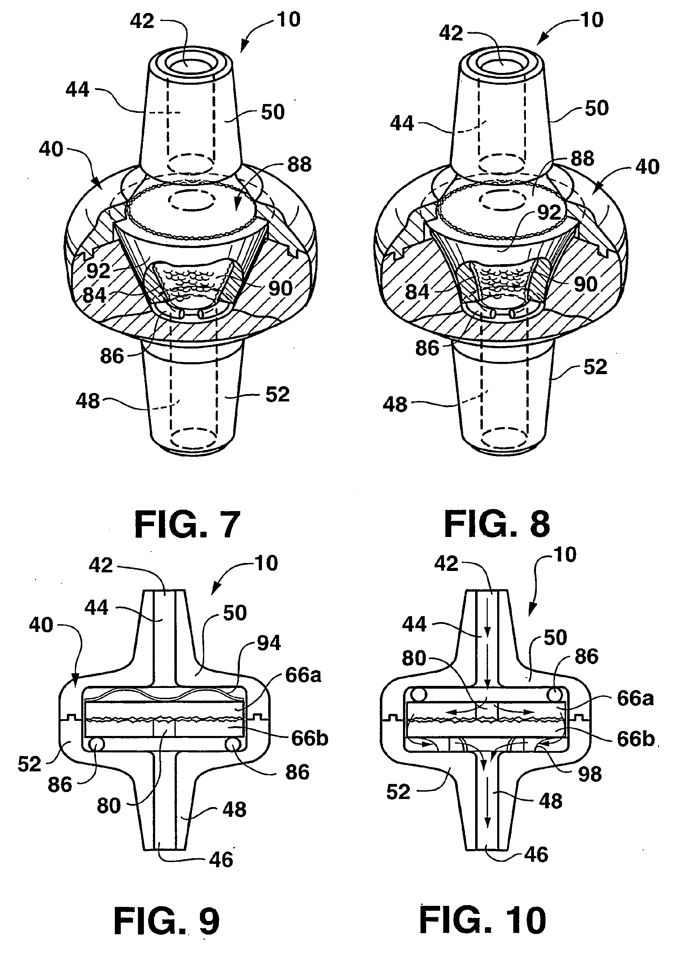

It should also be appreciated that the restriction devices 60 may take on various shapes and configurations. For example, in the embodiments of Figs. 7 and 8, conical restriction devices 88 are provided. A conical or truncated male member 90 having a rough outer surface is seated within a correspondingly shaped recess of an opposite member 92. The restrictive flow path is thus defined between the conical walls of the members 90 and 92, wherein at least one of these opposed surfaces defines a roughened surface. In the embodiment of Fig. 7, the conical opposed surfaces are relatively straight in that they have a constant slope. In the embodiment of Fig. 8, the opposed conical surfaces are curved, or have a radial component along at least a portion thereof. The embodiments of Figs. 7 and 8 provide for an increased surface area between the opposed surfaces defining the restrictive flow field 84, as compared to the flat disk devices 66a, 66b, of the prior embodiments.

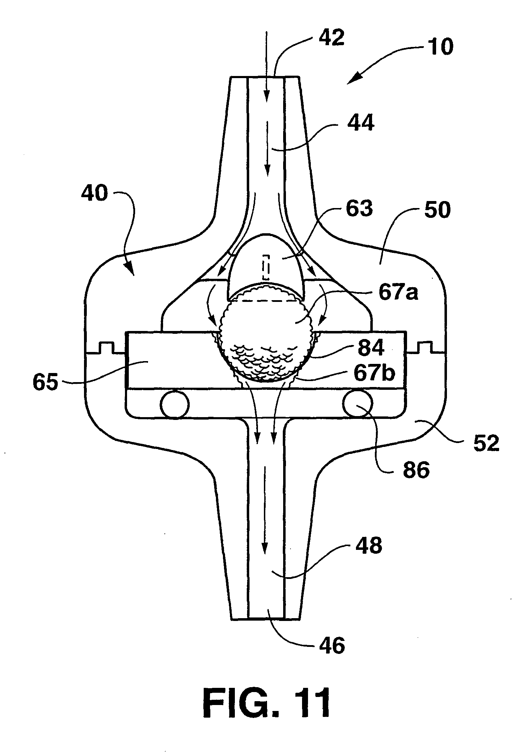

Fig. 11 illustrates an embodiment of a flow restrictor 10 wherein the restriction devices are defmed by a ball member 67a and a planar member 65 having a ball seat 67b defined therein. The restrictive fluid flow path 84 is defined between the circumferential portion of the ball member 67a and surface of the ball seat 67b. Either one or both of the ball surface or ball seat may be roughened. A flow distributor 63 may be incorporated within the housing as an integrally formed or separate component. The distributor 63 may take on any shape to direct fluid from the inlet passage 44 to the restrictive fluid flow path 84 and may also serve to positively engage and contain the ball 67a within the ball seat 67b. This embodiment may be desired from the standpoint of cost and ease of manufacture.

It should be appreciated that various configurations of restrictive devices may be derived empirically or otherwise by those skilled in the art to define a restrictive flow field between opposed surfaces in accordance with the principles of the present invention.

Fig. 9 illustrates an embodiment of a restrictor 10 incorporating a resilient biasing element within the housing 40. The biasing element may be in the form of a wave spring 94 as illustrated in the Fig. 9, or may be any other conventional biasing element such as a spring, and so forth. The biasing element serves to ensure that the restriction devices 66a, 66b are biased together so that the opposing surfaces properly define the desired restrictive flow path. It should also be appreciated that the sealing element 86 in the embodiments of Fig. 3a and Fig. 11, for example, may be formed of an elastomeric material and may also serve the function of biasing the restriction devices together.

Fig. 10 is an embodiment of a restrictor 10 wherein the fluid flows in a radially outward direction along the restrictive flow path between the restriction devices 66a, 66b. An orifice 80 is defined generally at the center of the upstream restriction device 66a. The sealing device (ring) 86 is disposed concentric about the orifice 80 such that fluid entering from inlet passage 44 is caused to flow through the orifice 80 to the restrictive flow path defined by the opposing surfaces of the restriction devices 66a, 66b. The fluid then flows radially outward along the flow path, as indicated by the arrows in Fig. 10, and around the periphery of the downstream restriction device 66b where it is directed to the outlet passage 48. Ridges 98, or any other suitable support structure, are provided within the housing to support the restriction device 66b and define a flow path for the fluid to the outlet passage 48.

It should be appreciated by those skilled in the art that modifications and variations may be made to the embodiments described above without departing from the scope of the invention. It is intended that the invention include these and other modifications as come within the scope of the appended claims and their equivalents.

## Claims

1. A medical apparatus flow restrictor (10), comprising:
a housing (40) having an inlet (42) and an outlet (46), and a fluid path defined through said housing (40) between said inlet (42) and said outlet (46);
at least one pair of opposed restriction devices (60) seated within said housing (40) between said inlet (42) and said outlet (46), said restriction devices comprising opposing surfaces (62, 64), said restriction devices (60) disposed in said flow path such that fluid delivered to said inlet (40) passes between said opposing surfaces (62, 64) prior to flowing from said outlet (46),
wherein said opposing surfaces (62, 64) have a relative degree of surface roughness and opposed surface area defined as a function of a desired flow rate of fluid through said restrictor,
**characterized in that** said opposing surfaces (62, 64) are placed in contact against each other such that the resulting flow is a function of the surface roughness.

2. The restrictor as in claim 1, wherein said restriction devices (60) comprise opposed flat planar members (66a, 66b) disposed within said housing (40) such that fluid from said inlet (42) flows radially between said opposing surfaces (62, 64) of said flat planar members (66a, 66b).

3. The restrictor as in claim 2, wherein a flow path is defined within said housing (40) such that the fluid flows around a perimeter (68) of said flat planar members (66a, 66b) and migrates radially inward between said opposing surfaces (62, 64) of said flat planar members (66a, 66b).

4. The restrictor as in claim 2, wherein a flow path is defined within said housing (40) such that the fluid flows through an orifice in upstream one of said flat planar members (66a, 66b) and then flows radially outward between said opposing surfaces (62, 64) of said flat planar members (66a, 66b).

5. The restrictor as in claim 1, wherein said restriction devices (60) are formed of a hard, con-compressible material such that fluid flow between said opposing surfaces (62, 64) is substantially constant regardless of a compressive pressure applied to said restriction devices (60).

6. The restrictor as in claim 1, wherein said restriction devices (60) are formed of a compressible material such that fluid flow between said opposing surfaces (62, 64) is varied by varying a compressive pressure applied to said restriction devices (60).

7. The restrictor as in claim 1, wherein said housing (40) comprises separate halves (50, 52), said restriction devices (60) placed within said halves (50, 52) prior to joining said halves (50, 52) to form said housing (40).

8. The restrictor as in claim 7, wherein said halves (50, 52) are separable after being joined for access to said restriction devices (60).

9. The restrictor as in claim 7, wherein said halves (50, 52) are permanently and non-separably joined.

10. The restrictor as in claim 1, further comprising a seal (86) disposed within said housing (40) relative to said restriction devices (60) to ensure that fluid flow through said restrictor (10) does not bypass said fluid flow path between said opposing surfaces (62, 64).

11. The restrictor as in claim 10, wherein at least one of said restriction devices (60) comprises an orifice (80) defining a flow path for the fluid into or out from said opposing surface (62, 64) of said restriction devices (60), said seal (86) disposed adjacent said restriction device (60) having said orifice (80).

12. The restrictor as in claim 1, wherein said restriction devices (60) comprise opposed flat discs (66a, 66b) disposed within said housing (40) such that fluid from said inlet (42) flows around a perimeter of said discs (66a, 66b) and migrates radially inward between said opposing surfaces (62, 64), said disc (66b) closest to said outlet (46) comprising an orifice (80) through which fluid flows from between said opposing surfaces (62, 64) to said outlet (46).

13. The restrictor as in claim 12, further comprising a sealing ring (86) disposed within said housing (40) between said outlet (46) and against an outer side of said disc (66b) closest to said outlet (46).

14. The restrictor as in claim 12, wherein said housing (40) comprises separate halves (50, 52), said discs (66a, 66b) placed within said halves (50, 52) prior to joining said halves (50, 52) to form said housing (40).

15. The restrictor as in claim 1, wherein said restriction devices (60) are disposed such that a plane between said opposing surfaces (62, 64) is generally perpendicular to an axis of said inlet (42) and said outlet (46).

16. The restrictor as in claim 1, wherein said restriction devices (60) are disposed such that a plane between said opposing surfaces (62, 64) is generally parallel to an axis of said inlet (42) and said outlet (46).

17. The restrictor as in claim 1, wherein said inlet (42) and said outlet (46) are connectable to tubing in fluid delivery system such that said restrictor (10) is placeable in-line within said system.

18. The restrictor as in claim 1, wherein said opposing surfaces (62, 64) are disposed in a generally flat plane essentially perpendicular to an axis of said inlet (42) and said outlet (46).

19. The restrictor as in claim 1, wherein said opposing surfaces (62, 64) are disposed in a generally conical plane between said inlet (42) and said outlet (46).

20. The restrictor as in claim 1, wherein said opposing surfaces (62, 64) are disposed in a generally curved plane between said inlet (42) and said outlet (46).

21. The restrictor as in claim 1, wherein said surface roughness of at least one of said opposing surfaces (62, 64) is defined in any one or combination of a controlled grinding, lapping, tumbling, sandblasting, or etching process.

22. The restrictor as in claim 1, wherein each of said opposing surfaces (62, 64) is roughened.

23. The restrictor as in claim 1, wherein only one of said opposing surfaces (62, 64) is roughened as compared to said other opposing surface (64, 62).

24. The restrictor as in claim 1, wherein said restriction devices (60) comprise a ball element (67a) seated within a ball seat (67b), said opposing surfaces (62, 64) defined by a circumferential portion of said ball element (67a) and said ball seat (67b).

25. The restrictor as in claim 1, further comprising a biasing element (94) disposed within said housing (40) so as to bias said restriction devices (60) together.

26. A medical fluid delivery system configured to deliver a fluid from a source to a patient at a regulated flow rate, said system comprising delivery tubing (30) and a medical apparatus flow restrictor (10) according to any of the preceding claims placed in-line in said tubing (30).

27. The fluid delivery system as in claim 26, wherein said flow restrictor (10) is disconnectable from said tubing (30).

28. The fluid delivery system as in claim 26, wherein said restriction devices (60) comprise opposed flat planar members (66a, 66b) disposed within said housing (40) such that fluid from said inlet (42) flows around a perimeter of said flat planar members and migrates radially inward between said opposing surfaces (62, 64) of said flat planar members (66a, 66b).

## Patentansprüche

1. Durchflussbegrenzer (10) für medizinische Geräte, umfassend:
ein Gehäuse (40), das einen Einlass (42) und einen Auslass (46) und
einen durch das Gehäuse (40) zwischen dem Einlass (42) und dem Auslass (46) festgelegten Fluid-Weg aufweist;
mindestens ein Paar gegenüberstehender Begrenzungsvorrichtungen (60), die innerhalb des Gehäuses (40) zwischen dem Einlass (42) und dem Auslass (46) gelegen sind, wobei die Begrenzungsvorrichtungen gegenüberstehende Flächen (62, 64) umfassen und die Begrenzungsvorrichtungen (60) so in dem Durchflussweg angeordnet sind, dass dem Einlass (40) zugeführtes Fluid vor dem Ausströmen aus dem Auslass (46) zwischen den gegenüberstehenden Flächen (62, 64) hindurchströmt,
wobei die gegenüberstehenden Flächen (62, 64) ein relatives Ausmaß an Oberflächenrauheit und an Fläche aufweisen, die als Funktion einer gewünschten Durchflussrate von Fluid durch den Begrenzer festgelegt sind,
**dadurch gekennzeichnet, dass** die gegenüberstehenden Flächen (62, 64) so in Kontakt gegeneinander gesetzt sind, dass der resultierende Durchfluss eine Funktion der Oberflächenrauheit ist.

2. Begrenzer nach Anspruch 1, wobei die Begrenzungsvorrichtungen (60) gegenüberstehende flache planare Elemente (66a, 66b) umfassen, die so in dem Gehäuse (40) angeordnet sind, dass Fluid von dem Einlass (42) radial zwischen den gegenüberstehenden Flächen (62, 64) der flachen planaren Elemente (66a, 66b) strömt.

3. Begrenzer nach Anspruch 2, wobei ein Durchflussweg innerhalb des Gehäuses (40) so festgelegt ist, dass das Fluid um einen Umfang (68) der flachen planaren Elemente (66a, 66b) strömt und radial nach innen zwischen den gegenüberstehenden Flächen (62, 64) der flachen planaren Elemente (66a, 66b) wandert.

4. Begrenzer nach Anspruch 2, wobei der Durchflussweg innerhalb des Gehäuses (40) so festgelegt ist, dass das Fluid durch eine Öffnung in dem stromaufwärts gelegenen der flachen planaren Elemente (66a, 66b) strömt und dann radial nach außen zwischen den gegenüberstehenden Flächen (62, 64) der flachen planaren Elemente (66a, 66b) strömt.

5. Begrenzer nach Anspruch 1, wobei die Begrenzungsvorrichtungen (60) aus einem harten, nicht kompressiblen Material ausgebildet sind, sodass die Fluid-Strömung zwischen den gegenüberstehenden Flächen (62, 64) im Wesentlichen konstant ist, unabhängig von einem auf die Begrenzungsvorrichtungen (60) einwirkenden zusammenpressenden Druck.

6. Begrenzer nach Anspruch 1, wobei die Begrenzungsvorrichtungen (60) aus einem kompressiblen Material ausgebildet sind, sodass die Fluid-Strömung zwischen den gegenüberstehenden Flächen (62, 64) durch Verändern eines auf die Begrenzungsvorrichtungen (60) einwirkenden zusammenpressenden Drucks variiert wird.

7. Begrenzer nach Anspruch 1, wobei das Gehäuse (40) getrennte Hälften (50, 52) umfasst, wobei die Begrenzungsvorrichtungen (60) in die Hälften (50, 52) gesetzt werden, bevor die Hälften (50, 52) verbunden werden, um das Gehäuses (40) auszubilden.

8. Begrenzer nach Anspruch 7, wobei die Hälften (50, 52) zum Zugang zu den Begrenzungsvorrichtungen (60) trennbar sind, nachdem sie verbunden sind.

9. Begrenzer nach Anspruch 7, wobei die Hälften (50, 52) dauerhaft und untrennbar verbunden sind.

10. Begrenzer nach Anspruch 1, weiter umfassend eine in dem Gehäuse (40) bezüglich der Begrenzungsvorrichtungen (60) angeordnete Dichtung (86), um sicherzustellen, dass Fluidströmung durch den Begrenzer (10) nicht an dem Fluid-Durchflussweg zwischen den gegenüberstehenden Flächen (62, 64) vorbeiströmt.

11. Begrenzer nach Anspruch 10, wobei mindestens eine der Begrenzungsvorrichtungen (60) eine Öffnung (80) umfasst, die einen Durchflussweg für das Fluid in die gegenüberstehende Fläche (62, 64) der Begrenzungsvorrichtungen (60) oder daraus heraus definiert, wobei die nahe der Begrenzungsvorrichtung (60) angeordnete Dichtung (86) die Öffnung (80) aufweist.

12. Begrenzer nach Anspruch 1, wobei die Begrenzungsvorrichtungen (60) gegenüberstehende flache Scheiben (66a, 66b) umfassen, die so in dem Gehäuse (40) angeordnet sind, dass Fluid von dem Einlass (42) um einen Umfang der Scheiben (66a, 66b) herum und radial nach innen zwischen den gegenüberstehenden Flächen (62, 64) wandert, wobei die dem Auslass (46) am nächsten befindliche Scheibe (66b) eine Öffnung (80) umfasst, durch die Fluid von zwischen den gegenüberstehenden Flächen (62, 64) zu dem Auslass (46) strömt.

13. Begrenzer nach Anspruch 12, weiter umfassend einen Dichtungsring (86), der innerhalb des Gehäuses (40) zwischen dem Auslass (46) und gegen eine Außenseite der dem Auslass (46) am nächsten befindlichen Scheibe (66b) angeordnet ist.

14. Begrenzer nach Anspruch 12, wobei das Gehäuse (40) getrennte Hälften (50, 52) umfasst, wobei die Scheiben (66a, 66b) in die Hälften (50, 52) gesetzt werden, bevor die Hälften (50, 52) verbunden werden, um das Gehäuse (40) auszubilden.

15. Begrenzer nach Anspruch 1, wobei die Begrenzungsvorrichtungen (60) so angeordnet sind, dass eine Ebene zwischen den gegenüberstehenden Flächen (62, 64) im Allgemeinen senkrecht zu einer Achse des Einlasses (42) und des Auslasses (46) steht.

16. Begrenzer nach Anspruch 1, wobei die Begrenzungsvorrichtungen (60) so angeordnet sind, dass eine Ebene zwischen den gegenüberstehenden Flächen (62, 64) im Allgemeinen parallel zu einer Achse des Einlasses (42) und des Auslasses (46) steht.

17. Begrenzer nach Anspruch 1, wobei der Einlass (42) und der Auslass (46) so mit dem Leitungsnetz in einem Fluid-Zufuhrsystem verbindbar sind, dass der Begrenzer (10) innerhalb des Systems in Reihe einfügbar ist.

18. Begrenzer nach Anspruch 1, wobei die gegenüberstehenden Flächen (62, 64) in einer im Allgemeinen flachen Ebene im Wesentlichen senkrecht zu einer Achse des Einlasses (42) und des Auslasses (46) stehen.

19. Begrenzer nach Anspruch 1, wobei die gegenüberstehenden Flächen (62, 64) in einer im Allgemeinen konischen Ebene zwischen dem Einlass (42) und dem Auslass (46) angeordnet sind.

20. Begrenzer nach Anspruch 1, wobei die gegenüberstehenden Flächen (62, 64) in einer im Allgemeinen gekrümmten Ebene zwischen dem Einlass (42) und dem Auslass (46) angeordnet sind.

21. Begrenzer nach Anspruch 1, wobei die Oberflächenrauheit mindestens einer der gegenüberstehenden Flächen (62, 64) in einem beliebigen oder in einer Kombination aus einem kontrollierten Schleif-, Läpp-, Trommel-, Sandstrahl- oder Ätzverfahren festgelegt ist.

22. Begrenzer nach Anspruch 1, wobei jede der gegenüberstehenden Flächen (62, 64) aufgeraut ist.

23. Begrenzer nach Anspruch 1, wobei nur eine der gegenüberstehenden Flächen (62, 64) im Vergleich zu der anderen gegenüberstehenden Fläche (64, 62) aufgeraut ist.

24. Begrenzer nach Anspruch 1, wobei die Begrenzungsvorrichtungen (60) ein Kugelelement (67a) umfassen, das in einem Kugelsitz (67b) sitzt, wobei die gegenüberstehenden Flächen (62, 64) durch einen Umfangsbereich des Kugelelements (67a) und den Kugelsitz (67b) definiert sind.

25. Begrenzer nach Anspruch 1, weiter umfassend ein in dem Gehäuse (40) angeordnetes Vorspannelement (94), um die Begrenzungsvorrichtungen (60) gegeneinander vorzuspannen.

26. Medizinisches Fluid-Zufuhrsystem, das gestaltet ist, ein Fluid von einer Quelle zu einem Patienten mit einer geregelten Durchflussrate zuzuführen, wobei das System Zufuhrleitungen (30) und einen Durchflussbegrenzer (10) für medizinische Geräte nach einem der vorhergehenden Ansprüche umfasst, der in Reihe in die Zufuhrleitungen (30) eingefügt ist.

27. Fluid-Zufuhrsystem nach Anspruch 26, wobei der Durchflussbegrenzer (10) von den Leitungen (30) trennbar ist.

28. Fluid-Zufuhrsystem nach Anspruch 26, wobei die Begrenzungsvorrichtungen (60) gegenüberstehende flache planare Elemente (66a, 66b) umfassen, die so in dem Gehäuse (40) angeordnet sind, dass Fluid von dem Einlass (42) um einen Umfang der flachen planaren Elemente herum strömt und radial nach innen zwischen den gegenüberstehenden Flächen (62, 64) der flachen planaren Elemente (66a, 66b) wandert.

## Revendications

1. Réducteur de débit pour un appareil médical (10), comprenant:
un boîtier (40) comportant un orifice d'admission (42), un orifice de sortie (46) et un circuit de fluide défini à travers ledit boîtier (40) entre ledit orifice d'admission (42) et ledit orifice de sortie (46);
au moins une paire de dispositifs réducteurs opposés (60) logés au sein dudit boîtier (40) entre ledit orifice d'admission (42) et ledit orifice de sortie (46), lesdits dispositifs réducteurs comprenant des surfaces opposées (62, 64), lesdits dispositifs réducteurs (60) étant disposés dans ledit circuit de fluide de telle manière que le fluide délivré audit orifice d'admission (40) circule entre lesdites surfaces opposées (62, 64) avant de s'écouler dudit orifice de sortie (46),
dans lequel lesdites surfaces opposées (62, 64) présentent un degré relatif de rugosité de surface et de dimension des surfaces opposées définie comme une fonction d'un débit de fluide souhaité à travers ledit réducteur,
**caractérisé en ce que** lesdites surfaces opposées (62, 64) sont placées en contact l'une contre l'autre de telle manière que le débit résultant soit une fonction de la rugosité de surface.

2. Réducteur selon la revendication 1, dans lequel lesdits dispositifs réducteurs (60) comportent des membres planaires plats opposés (66a, 66b) disposés au sein dudit boîtier (40) de telle manière que le fluide provenant dudit orifice d'admission (42) s'écoule radialement entre lesdites surfaces opposées (62, 64) desdits membres planaires plats (66a, 66b).

3. Réducteur selon la revendication 2, dans lequel un circuit de fluide est défini au sein dudit boîtier (40) de telle manière que le fluide s'écoule autour d'un périmètre (68) desdits membres planaires plats (66a, 66b) et migre radialement vers l'intérieur, entre lesdites surfaces opposées (62, 64) desdits membres planaires plats (66a, 66b).

4. Réducteur selon la revendication 2, dans lequel un circuit de fluide est défini au sein dudit boîtier (40) de telle manière que le fluide circule à travers un orifice dans le membre en amont parmi lesdits membres planaires plats (66a, 66b), puis circule radialement vers l'extérieur, entre lesdites surfaces opposées (62, 64) desdits membres planaires plats (66a, 66b).

5. Réducteur selon la revendication 1, dans lequel les dispositifs réducteurs (60) sont constitués d'un matériau dur incompressible, tel que l'écoulement de fluide entre lesdites surfaces opposées (62, 64) soit essentiellement constant, indépendamment d'une pression de compression appliquée auxdits dispositifs réducteurs (60).

6. Réducteur selon la revendication 1, dans lequel les dispositifs réducteurs (60) sont constitués d'un matériau compressible, tel que l'écoulement de fluide entre lesdites surfaces opposées (62, 64) soit variée par variation d'une pression de compression appliquée auxdits dispositifs réducteurs (60).

7. Réducteur selon la revendication 1, dans ledit boîtier (40) comprend des moitiés séparées (50, 52), lesdits dispositifs réducteurs (60) étant placés au sein desdites moitiés (50, 52) avant la jonction desdites moitiés (50, 52) pour former ledit boîtier (40).

8. Réducteur selon la revendication 7, dans lequel lesdites moitiés (50, 52) sont séparables après avoir été jointes pour l'accès auxdits dispositifs réducteurs (60).

9. Réducteur selon la revendication 7, dans lequel lesdites moitiés (50, 52) sont jointes de manière permanente et non-séparable.

10. Réducteur selon la revendication 1, comportant en outre un joint (86) disposé au sein dudit boîtier (40) relativement auxdits dispositifs réducteurs (60) pour assurer que l'écoulement de fluide à travers ledit réducteur (10) ne fait pas dériver ledit circuit d'écoulement de fluide entre lesdites surfaces opposées (62, 64).

11. Réducteur selon la revendication 10, dans lequel l'un au moins desdits dispositifs réducteurs (60) comporte un orifice (80) définissant une voie de passage pour le fluide dans ou depuis ladite surface opposée (62, 64) desdits dispositifs réducteurs (60), ledit joint (86) étant disposé de manière adjacente audit dispositif réducteur (60) possédant ledit orifice (80).

12. Réducteur selon la revendication 1, dans lequel lesdits dispositifs réducteurs (60) comportent des disques plats opposés (66a, 66b) disposés au sein dudit boîtier (40) de telle manière que le fluide provenant dudit orifice d'admission (42) s'écoule autour d'un périmètre desdits disques (66a, 66b) et migre radialement vers l'intérieur entre lesdites surfaces opposées (62, 64), ledit disque (66b) le plus proche dudit orifice de sortie (46) comportant un orifice (80) à travers lequel s'écoule le fluide depuis l'intervalle entre lesdites surfaces opposées (62, 64) vers ledit orifice de sortie (46).

13. Réducteur selon la revendication 12, comportant en outre une bague d'étanchéité (86) disposée au sein dudit boîtier (40) entre ledit orifice de sortie (46) et contre une face extérieure dudit disque (66b) le plus proche dudit orifice de sortie (46).

14. Réducteur selon la revendication 12, dans ledit boîtier (40) comprend des moitiés séparées (50, 52), lesdits disques (66a, 66b) étant placés au sein desdites moitiés (50, 52) avant la jonction desdites moitiés (50, 52) pour former ledit boîtier (40).

15. Réducteur selon la revendication 1, dans lequel lesdits dispositifs réducteurs (60) sont disposés de telle manière qu'un plan entre lesdites surfaces opposées (62, 64) soit généralement perpendiculaire à un axe dudit orifice d'admission (42) et dudit orifice de sortie (46).

16. Réducteur selon la revendication 1, dans lequel lesdits dispositifs réducteurs (60) sont disposés de telle manière qu'un plan entre lesdites surfaces opposées (62, 64) soit généralement parallèle à un axe dudit orifice d'admission (42) et dudit orifice de sortie (46).

17. Réducteur selon la revendication 1, dans lequel ledit orifice d'admission (42) et ledit orifice de sortie (46) sont connectables pour le tubage dans un système de livraison de fluide, si bien que ledit réducteur (10) peut être placé en ligne au sein dudit système.

18. Réducteur selon la revendication 1, dans lequel lesdites surfaces opposées (62, 64) sont disposées dans un plan généralement plat essentiellement perpendiculaire à un axe dudit orifice d'admission (42) et dudit orifice de sortie (46).

19. Réducteur selon la revendication 1, dans lequel lesdites surfaces opposées (62, 64) sont disposées dans un plan généralement conique entre ledit orifice d'admission (42) et ledit orifice de sortie (46).

20. Réducteur selon la revendication 1, dans lequel lesdites surfaces opposées (62, 64) sont disposées dans un plan généralement curviligne entre ledit orifice d'admission (42) et ledit orifice de sortie (46).

21. Réducteur selon la revendication 1, dans lequel ladite rugosité de surface de l'une au moins desdites surfaces opposées (62, 64) est définie dans l'un quelconque ou une combinaison des processus de rectification, rodage, tonnelage, sablage ou décapage.

22. Réducteur selon la revendication 1, dans lequel chacune desdites surfaces opposées (62, 64) est rendue rugueuse.

23. Réducteur selon la revendication 1, dans lequel l'une seulement desdites surfaces opposées (62, 64) est rendue rugueuse en comparaison avec ladite autre surface opposée (64, 62).

24. Réducteur selon la revendication 1, dans lequel lesdits dispositifs réducteurs (60) comportent un élément en boule (67a) logés au sein d'une cage de boule (67b), lesdites surfaces opposées (62, 64) étant définies par une partie circonférentielle dudit élément en boule (67a) et de ladite cage de boule (67b).

25. Réducteur selon la revendication 1, comportant en outre un élément de retenue (94) disposé au sein dudit boîtier (40) de manière à retenir lesdits dispositifs réducteurs (60) l'un contre l'autre.

26. Système de livraison de fluide médical configuré pour délivrer, depuis une source, un fluide à un patient à un débit d'écoulement régulé, ledit système comportant un tubage de livraison (30) et un réducteur (10) de débit d'écoulement d'appareil médical selon l'une quelconque des revendications précédentes, placé en ligne dans ledit tubage (30).

27. Système de livraison de fluide selon la revendication 26, dans lequel ledit réducteur de débit (10) peut être déconnecté dudit tubage (30).

28. Système de livraison de fluide selon la revendication 26, dans lequel lesdits dispositifs réducteurs (60) comportent des membres planaires plats opposés (66a, 66b) disposés au sein dudit boîtier (40) de telle manière le fluide provenant dudit orifice d'admission (42) s'écoule autour d'un périmètre desdits membres planaires plats et migre radialement vers l'intérieur, entre lesdites surfaces opposées (62, 64) desdits membres planaires plats (66a. 66b).
